# EUROPÄISCHE PATENTANMELDUNG

(11) **EP 1 447 043 A1**
(43) Veröffentlichungstag der Anmeldung: **18.08.2004**
(21) Anmeldenummer: 03028398.0
(22) Anmeldetag: 11.12.2003
(51) Int. Cl.: A61B 1/04, A61B 5/00, G01J 3/44

(54) **Vorrichtung zur biologischen Diagnose von Gewebe**

(30) Priorität: 11.02.2003 DE 10305599
(71) Anmelder: Richard Wolf GmbH, 75438 Knittlingen (DE)
(72) Erfinder: Weber, Bernd Claus, Dr. Dipl.-Ing., 76228 Karlsruhe (DE); Goll, Thomas, 75438 Knittlingen (DE); Dolt, Martin, Dipl.-Phys., 75438 Knittlingen (DE); Müller, Stefan, Dipl.-Ing., 75015 Bretten-Dieselsheim (DE); Eidner, Philip, 75015 Bretten-Büchig (DE); Pereira-Delgado, Nicolas, 75433 Maulbronn (DE); von den Bergh, Hubert, Prof. Dr., 1025 St-Sulpice (CH); Wagnieres, Georg, Dr., 1095 Lutry (CH); Glanzmann, Thomas, Dr., 4102 Binningen (CH); Gabrecht, Tanja, 58739 Wickede (DE)
(74) Vertreter: Wilcken, Thomas, Dipl.-Ing.

(57) **Zusammenfassung**

Es wird eine Vorrichtung zur bildgebenden Diagnose von Gewebe (1) unter wahlweiser Anwendung von mindestens zwei Diagnosemethoden beschrieben, nämlich einem Modus zur diagnostischen Weißlicht-Endoskopie DWLE und einem Modus zur diagnostischen Autofluoreszenz-Endoskopie DAFE bzw. DAFE I. Optional gibt es eine dritte Diagnosemethode, nämlich einen zweiten Modus zur diagnostischen Autofluoreszenz-Endoskopie DAFE II. Die Vorrichtung besteht aus einer Lichtquelle (2) mit einem oder mehreren Leuchtmitteln, deren Licht über einen Lichtleiter (3) dem Gewebe (1) zugeführt wird. Das Bild des zu untersuchenden Gewebes wird mittels einer Bildübertragungseinheit (4) auf eine Bildaufnahmeeinheit (5) abgebildet, und die dort erzeugten Bildsignale werden in einer Bildverarbeitungseinheit (6) aufbereitet und auf einem Monitor (7) dargestellt. Alternativ kann das Bild auch direkt über eine Bildübertragungseinheit dem menschlichen Auge zugeführt werden. Zur Steigerung der Sensitivität von prä- und frühmalignen Läsionen gegenüber gesundem Gewebe und zur Steigerung der Spezifität von prä- und frühmalignen Läsionen gegenüber nichtmalignen Gewebeatypien wird im ersten Modus der diagnostischen Autofluoreszenz-Endoskopie DAFE bzw. DAFE I das spektrale Band der Strahlung für die Fluoreszenz-Anregung auf einen schmalen Bereich um 405 nm eingegrenzt (Fig. 12).

## Beschreibung

Die Erfindung betrifft eine Vorrichtung zur bildgebenden Diagnose von Gewebe unter wahlweiser Anwendung von zwei oder drei Diagnosemethoden, nämlich einem Arbeitsmodus zur diagnostischen Weißlicht-Endoskopie DWLE, einem ersten Arbeitsmodus zur diagnostischen Autofluoreszenz-Endoskopie DAFE bzw. DAFE I und gegebenenfalls einem zweiten Arbeitsmodus zur diagnostischen Autofluoreszenz-Endoskopie DAFE II, mit einer Lichtquelle, deren Licht über ein Endoskop zum Gewebe geleitet wird, und mit einer Bildübertragungseinheit und einer Bildaufnahmeeinheit, welche mit einer Bildverarbeitungseinheit verbunden ist, über welche ein Monitor mit einem Bildsignal versorgt wird. Die Bildüberbtragungseinheit kann auch direkt mit dem menschlichen Auge in Verbindung stehen. In diesem Fall kann auf die Bildaufnahmeeinheit, die Bildverarbeitungseinheit und den Monitor verzichtet werden.

Die DAFE ist mittlerweile zu einem etablierten Verfahren bei der Lokalisierung und Detektion von prä- und frühmalignen Läsionen geworden, die einerseits aufgrund ihres geringen Malignitätsgrades noch mit sehr hoher Wahrscheinlichkeit kurativ therapiert werden können, die aber andererseits bei der DWLE aufgrund ihrer geringen Größe und aufgrund ihrer topographischen Unauffälligkeit kaum oder gar nicht sichtbar sind.

Bei der DAFE wird menschliches Gewebe, z. B. Gewebe der Bronchialschleimhaut, mit Strahlung aus dem ultravioletten und / oder violetten und / oder blauen Spektralbereich angeregt, um eine Autofluoreszenz im längerwelligen sichtbaren Spektralbereich, vor allen Dingen im Grünen und Roten, zu erzeugen. Eine Gewebedifferenzierung wird dadurch möglich, dass gesundes Gewebe eine starke Fluoreszenz aufweist, während mit zunehmendem Grad der Gewebeatypie und mit zunehmendem Malignitätsgrad die Intensität der Fluoreszenz abnimmt. Außerdem ist der Intensitätsrückgang im Grünen stärker als im Roten. Daraus folgt, dass bei gewöhnlicher bildgebender Darstellungsweise pathologisch verändertes Gewebe zum einen dunkler und zum anderen rötlicher erscheint als gesundes Gewebe.

Die bekannten Autofluoreszenz-Systeme zeichnen sich allesamt durch eine im Vergleich zur DWLE stark verbesserte Sensitivität für präinvasive, d. h. intraepitheliale Läsionen (Dysplasien und Carcinoma in situ, nachfolgend auch als "prä- und frühmaligne Läsionen" oder "prä- und frühmaligne Gewebeatypien" bezeichnet) gegenüber gesundem Gewebe aus.

Problematischer hingegen verhält es sich bei diesen Systemen mit der Spezifität für diese Läsionen: Entzündetes Gewebe und metaplastisch verändertes Gewebe, beides Gewebeformen, welche nicht oder noch nicht als prä- oder frühmaligne eingestuft werden und daher nachfolgend auch unter dem Begriff "nichtmaligne Gewebeatypien" zusammengefaßt werden, lassen sich im Autofluoreszenzbild mit den bekannten Vorgehensweisen praktisch nicht von Dysplasien und Carcinoma in situ, also von den bereits prä- und frühmalignen Läsionen, deren Detektion deshalb im Hinblick auf die Prognose des Patienten von größtem Interesse ist, unterscheiden. Um aber eine gesicherte und endgültige Beurteilung abgeben und eine (Prä-) Malignität ausschließen zu können, muss von allen Stellen, die im Autofluoreszenzbild auffällig sind, also sowohl von den prä- und frühmalignen als auch von den entzündeten und metaplastischen, eine Biopsie gemacht werden und diese dem Pathologen zugeführt werden. Die Vielzahl von Probeentnahmen bedeutet aber einen höheren Zeitaufwand bei der Endoskopie und damit in direktem Zusammenhang stehende höhere Kosten, außerdem eine stärkere Belastung für den Patienten sowie weitere Kosten für die Untersuchung der nicht-karzinomatösen Bioptate durch den Pathologen.

Dieser an die bisherigen Autofluoreszenz-Systeme und deren Funktionsweise geknüpfte, gravierende Nachteil verlangt nach einer Vorgehensweise und einem System mit einer höheren Spezifität für Dysplasien und Carcinoma in situ, also für die intraepithelialen, prä- und frühmalignen Läsionen gegenüber den nichtmalignen Gewebeatypien bei unverändert guter Sensitivität für diese Läsionen gegenüber gesundem Gewebe. Das bedeutet, dass diese Läsionen bereits während der endoskopischen Untersuchung, also schon im Autofluoreszenzbild, klar von den nichtmalignen Metaplasien und Entzündungen unterscheidbar sein müssen, so dass auf "unnötige", patientenbelastende, kostensteigernde, die Untersuchungszeit verlängernde Biopsien an lediglich entzündetem und metaplastischem Gewebe weitgehend verzichtet werden kann.

Gleichzeitig soll die Sensitivität für die prä- und frühmalignen Läsionen gegenüber gesundem Gewebe maximal sein, d. h. pathologisch verändertes Gewebe soll im Bild dadurch stark auffällig sein, dass es sich bei der gewählten Vorgehensweise mit hohem Farb- und / oder Helligkeitskontrast vom umgebenden gesunden Gewebe abhebt.

Optional soll die Möglichkeit bestehen, meta plastisch verändertes Gewebe sowohl von pathologisch unverändertem Gewebe, also gesundem Gewebe, als auch von entzündetem Gewebe differenzieren zu können. Die Detektion von Metaplasien kann beispielsweise bei der Entscheidung, chemopräventive Maßnahmen zu treffen, von Bedeutung sein.

Das System soll desweiteren uneingeschränkt tauglich sein für die DWLE. Das bedeutet u. a., dass die Bildqualität gegenüber vergleichbaren konventionellen endoskopischen Systemen unverändert gut sein muss. Diesbezüglich ist vor allen Dingen folgendes zu berücksichtigen: Die Intensität der Autofluoreszenz ist um etwa einen Faktor hundert geringer als die Intensität des am Gewebe remittierten, also gerichtet reflektierten und zurückgestreuten Fluoreszenz-Anregungslichtes. Um dennoch die Autofluoreszenz sichtbar zu machen, muss das die Autofluoreszenz dominierende, am Gewebe remittierte Fluoreszenz-Anregungslicht im Bildpfad, also auf dem Weg vom Gewebe zum bilderfassenden Sensorsystem, völlig oder zumindest zum allergrößten Teil geblockt werden.

Dies geschieht in der Regel über ein in diesen Bildpfad eingebrachtes optisches Hochpassfilter, welches dadurch charakterisiert ist, dass seine Transmission im Fluoreszenz-Anregungsband weitgehend bei Null liegt, während seine Transmission im langwellligen spektralen Bereich jenseits des Fluoreszenz-Anregungsbandes, also im Bereich der Autofluoreszenz und vor allen Dingen in jenem Bereich, wo die Unterschiede der gewebezustandsspezifischen Autofluoreszenz am größten sind, hoch ist, idealerweise sogar gegen hundert Prozent geht. Liegt das Fluoreszenz-Anregungsband im Sichtbaren, dann muss entsprechend den vorangehenden Ausführungen im Bildpfad mindestens dieser Anteil des sichtbaren Lichtes bzw. der größte Anteil davon geblockt werden. Verwendet das endoskopische System Videoendoskope, dann ist der Einfachheit halber aus technisch-konstruktiven Gründen das optische Blockfilter permanent im Bildpfad eingebracht. Soll das endoskopische System und insbesondere das Video-Endoskop auch für die DWLE verwendet werden, dann fehlen die durch das fest installierte optische Blockfilter permanent geblockten, kurzwelligen Strahlungsanteile des sichtbaren Spektralbereiches im Weißlichtbild. Wenn jedoch Anteile des Sichtbaren im Weißlichtbild fehlen, lassen sich beispielsweise bestimmte Farben nicht mehr oder kaum noch voneinander unterscheiden. Daraus resultierend kann es zu einer Beeinträchtigung bei der Gewebedifferenzierung kommen, und das System kann nicht mehr als uneingeschränkt weißlichttauglich bezeichnet werden,

Außerdem soll ein einfacher und schneller Wechsel zwischen dem Weißlicht-Modus und dem Autofluoreszenz-Modus möglich sein. Das bedeutet, dass dieser Wechsel über einen Tastendruck am Endoskop oder an einem der zugehörigen Geräte, das Betätigen eines Fußschalters oder über Sprachsteuerung realisierbar ist. Ein Austausch von Komponenten soll nicht erforderlich sein.

Weiterhin muss das System preiswert sein und insbesondere das Bedienund Anwendungsteil, also entweder das Video-Endoskop oder alternativ das konventionelle Endoskop mit aufgesetzter Kamera, handlich und leicht. Das bedingt u. a. einen einfachen Aufbau des Bedien- und Anwendungsteils und erfordert außerdem, dass beides, die DWLE und die DAFE, mit einem Sensor (1-Chip) oder einer Sensorgruppe (3-Chip) realisiert werden.

Und schließlich soll es möglich sein, beim Endoskop und dessen Aufbau auf konventionelle optische Komponenten zurückgreifen zu können, also beispielsweise auf solche mit erhöhter UV-Transmission verzichten zu können, da dies in der Regel anderweitig mit Nachteilen verbunden ist, auf die noch später eingegangen wird.

Es wurden unterschiedliche Wege aufgezeigt, um zumindest teilweise den obigen Forderungen gerecht zu werden. Ein Schwerpunkt liegt dabei oft auf der Spezifizierung des Wellenlängen bereiches für die Fluoreszenz-Anregung.

In der DE 101 36 419 A 1 ist das Wellenlängenband für die Fluoreszenz-Anregung ins UV gelagert. Da bei der dort vorgestellten Vorgehensweise zwei Strahlungsquellen zum Einsatz kommen, nämlich eine für die DWLE und die andere für die DAFE, und die optischen Achsen der Strahlenbündel der beiden Strahlungsquellen überlagert werden sollen, um deren abgegebene Strahlung sequenziell in ein gemeinsames Faserbündel einzukoppeln, und da außerdem bei der DWLE das Gewebe idealerweise mit Licht aus dem gesamten sichtbaren Spektralbereich beleuchtet werden soll, um eine optimale Farbwiedergabe und in dieser Hinsicht ein optimales Weißlichtbild zu erzielen, ist die Positionierung des Fluoreszenz-Anregungsbandes in das dem sichtbaren Licht benachbarte UV sinnvoll, zumindest bei der gewählten Form des Lichtprojektoraufbaus, bei welcher ein 45°-Hochpassfilter verwendet wird, um die beiden Strahlenbündel der beiden Lichtquellen zu überlagern. Allerdings bringt diese Vorgehensweise große Nachteile mit sich, vor allen Dingen in medizinisch-diagnostischer Hinsicht.

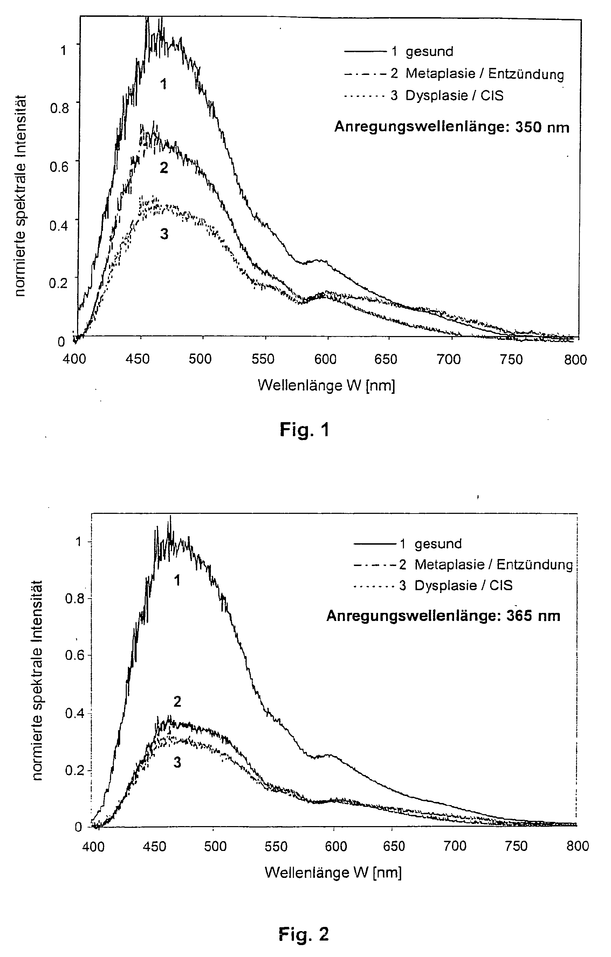

Zur Erläuterung sei auf die Fig. 1 bis 4 der anliegenden Zeichnung verwiesen. Diese zeigen das spektrale Fluoreszenzverhalten von menschlichem Bronchialgewebe für verschiedene, eng nebeneinander liegende, äquidistante Fluoreszenz-Anregungswellenlängen im UV für unterschiedliche Gewebezustände, d. h. für gesundes Gewebe und diverse Formen der Gewebeatypie. In diesen und auch bei den später gezeigten Schaubildern bedeutet "CIS" Carcinoma in situ. Die spektralen Fluoreszenzintensitäten sind jewels auf das Fluoreszenzmaximum von gesundem Gewebe normiert. In Bezug auf die eingangs angesproche Problematik werden aus den Kurvenverläufen zwei Dinge ersichtlich.

Erstens: In dem Wellenlängenbereich, wo die Fluoreszenz am stärksten ist, und welcher dementsprechend auch das Autofluoreszenzbild am stärksten prägt, also etwa zwischen 450nm und 500nm, ist die spektrale Fluoreszenzintensität für gesundes Gewebe nur um einen Faktor zwei bis drei höher als die spektrale Fluoreszenzintensität für Dysplasien und Carcinoma in situ. Eine Ausnahme bildet hier der Übergang vom UV ins Sichtbare: Bei einer Anregungswellenänge von 395nm ist dieser Faktor etwas größer als fünf. Folglich werden Dysplasien und Carcinoma in situ bei einer Fluoreszenz-Anregung mit Strahlung aus dem UV im Autofluoreszenzbild nur geringfügig, zumindest aber nicht in der maximal möglichen Form dunkler erscheinen als das umgebende gesunde Gewebe. Dies resultiert in einer Sensitivität für prä- und frühmaligne Läsionen, die nicht optimal ist.

Zweitens: Im gleichen betrachteten und den das Autofluoreszenzbild dominierenden Emissions-Wellenlängenbereich, also zwischen 450 nm und 500 nm, sind die spektralen Fluoreszenzintensitäten bei Anregungswellenlängen im UV, insbesondere bei den Anregungswellenlängen 365 nm, 380 nm und 395 nm, für metaplastisches und entzündetes Gewebe vergleichbar denjenigen für Dysplasien und Carcinoma in situ bezogen auf die spektralen Fluoreszenzintensitäten von gesundem Gewebe. Das bedeutet, dass die prä- und frühmalignen Läsionen von atypischem aber nichtmalignem Gewebe praktisch nicht differenziert werden können; alle Formen der Gewebeatypien, maligne und nichtmaligne, erscheinen nämlich ähnlich dunkel im Vergleich zum umgebenden hellen gesunden Gewebe. Dies wiederum resultiert in einer schlechten Spezifität für prä- und frühmaligne Läsionen gegenüber den nichtmalignen Gewebeatypien.

Aber auch in technischer Hinsicht bringt die in der DE 101 36 419 A 1 dargestellte Vorgehensweise Probleme. Bekanntlich ist die Autofluoreszenz von menschlichem Gewebe generell von geringer Intensität, so dass alle erdenklichen Maßnahmen getroffen werden müssen, um ausreichend helle Fluoreszenzbilder zu erzeugen. Eine dieser Maßnahmen ist die Bereitstellung eines Maximums an Fluoreszenz-Anregungslicht, welches dann auch am Gewebe ankommen muss, um dort ein Maximum an Autofluoreszenz erzeugen zu können. Um dies zu gewährleisten, ist u. a. eine gute Transmission der optischen Komponenten, welche das Anregungslicht von der Lichtquelle zum Gewebe übertragen, erforderlich. Dies ist bei konventionellen Linsen und vor allen Dingen bei gewöhnlichen Glasfasern nicht gegeben, wenn es sich bei der Strahlung für die Fluoreszenz-Anregung um UV-Strahlung handelt. Die konventionellen Glasfaserbündel zeichnen sich aus durch einen starken Rückgang der Transmission beim Übergang vom Sichtbaren ins UV. Eine Alternative sind theoretisch Quarzfasern, welche durch eine deutlich bessere Transmission im Kurzwelligen charakterisiert sind. Nachteil hierbei ist jedoch die geringe numerische Apertur dieser Fasern, welche Probleme bei der proximalen Einkopplung der Strahlung in das Faserbündel verursacht und damit das Problem nur verlagert.

In der DE 198 49 777 A 1 wird ein Autofluoreszenz-System beschrieben, dessen Fluoreszenz-Anregungsfilter so spezifiziert ist, dass die Fluoreszenz-Anregung in einem Wellenlängenband zwischen 425 nm und 455 nm vorgenommen wird. Der Grund für diese Vorgehensweise liegt in der Annahme, dass das körpereigene Molekül Riboflavin bzw. dessen Fluoreszenzverhalten maßgeblich für die Möglichkeit der Gewebedifferenzierung verantwortlich ist und dass die Fluoreszenztätigkeit von Riboflavin bei einer Anregungswellenlänge von 440 nm am stärksten ist. Die Verbreiterung des Anregungsbandes um jeweils 15 nm zum Kürzerwelligen und zum Längerwelligen wird vorgenommen, um das Gewebe mit mehr Anregungslicht zu beaufschlagen und um dadurch eine stärkere Fluoreszenzintensität zu erzielen.

Allerdings wird man auch bei dieser Vorgehensweise mit zwei Problemen konfrontiert: Das im Bildpfad erforderliche Blockfilter, also das optische Hochpassfilter, welches dafür sorgt, dass kein vom Gewebe remittiertes Fluoreszenz-Anregungslicht das Sensorsystem erreichen kann, blockt einen breiten spektralen Bereich im Blauen, nämlich das gesamte Licht mit einer Wellenlänge von weniger als 480 nm. Um dennoch eine uneingeschränkte Weißlichttauglichkeit des Systems zu gewährleisten, wird deshalb für die DWLE ein zweites Sensorsystem ohne vorgelagertes Blockfilter verwendet. Das macht das System jedoch teuerer und außerdem das Bedien- und Anwendungsteil größer, schwerer und unhandlicher.

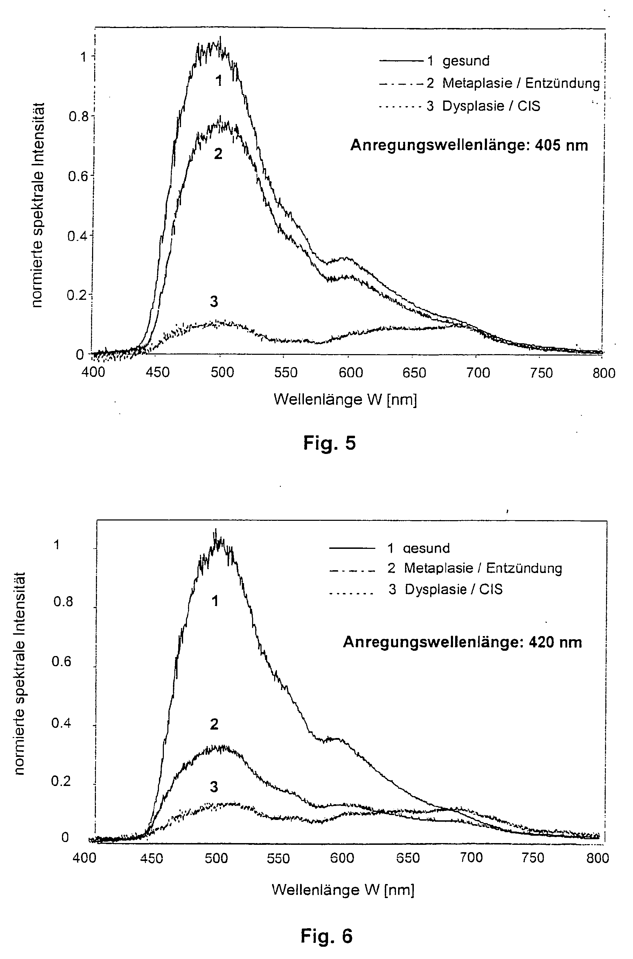

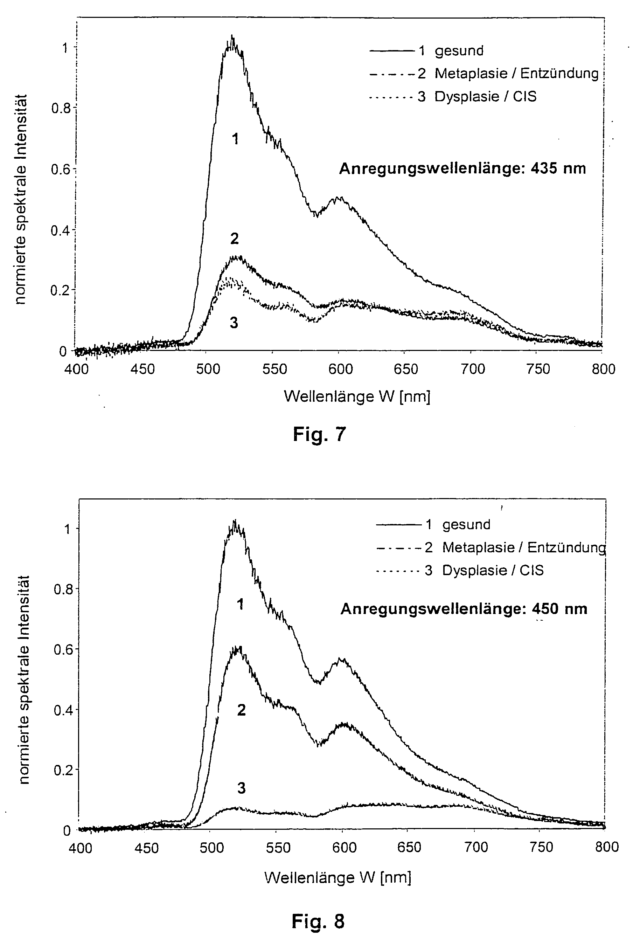

Des Weiteren wird aus den Fig. 6 und 7 der anliegenden Zeichnung deutlich, dass auch hier wieder mit Anregungswellenlängen gearbeitet, bei welchen die resultierenden spektralen Fluoreszenzintensitäten für Dysplasien und Carcinoma in situ im Vergleich zu denjenigen von gesundem Gewebe ähnlich gering sind wie die spektralen Fluoreszenzintensitäten von meta plastischem Gewebe und Entzündungen. Dies resultiert, ähnlich der Situation im vorhergehend beschriebenen System, in einer unzureichenden Spezifität für prä- und frühmaligne Läsionen gegenüber den nichtmalignen Gewebeveränderungen.

Prinzipiell vergleichbar mit dem System gemäß der DE 198 49 777 A 1 ist der Aufbau im US-Patent 6 099 466: Dort ist ein weißlichttaugliches Autofluoreszenzsystem mit Videoendoskop beschrieben. Das Fluoreszenz-Anregungsband ist bei diesem System sogar auf 480 nm ausgedehnt. Das Blockfilter im Bildpfad lässt dementsprechend erst Licht oberhalb von 520 nm durch. Da für ein gutes Weißlichtbild nicht auf den gesamten spektralen Bereich unterhalb dieser Wellenlänge verzichtet werden kann, ist am distalen Ende des Videoendoskops, also am Einführungsteil, ein weiteres Sensorsystem ohne Blockfilter untergebracht, welches beim Umschalten in die DWLE aktiviert wird.

Nachteilig bei dieser Vorgehensweise ist jedoch, dass bei ohnehin schlechten Platzverhältnissen im Einführteil des Endoskops ein zweites Sensorsystem untergebracht werden muss. Dies geht auf Kosten des Arbeitskanals oder des Beleuchtungs- bzw. Fluoreszenz-Anregungskanals. Gerade aber im Beleuchtungs- bzw. Fluoreszenz-Anregungskanal muss Platz für genügend viele Beleuchtungsfasern bereitgestellt werden, da sonst nicht ausreichend Fluoreszenz-Anregungslicht zur Erzeugung heller Autofluoreszenzbilder an das Gewebe herangeführt werden kann. Wird hingegen der Arbeitskanal zu klein gewählt, geht dies auf Kosten der Qualität der Probeentnahmen.

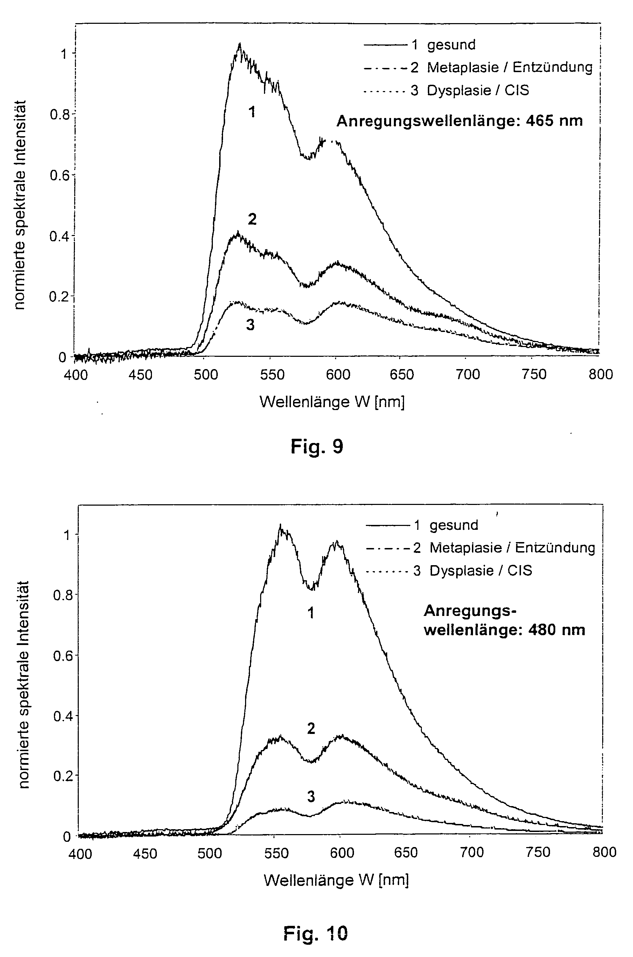

Außerdem liefert auch dieses System mit seiner breitbandigen Fluoreszenz-Anregung wie auch schon die vorausgehend beschriebenen Systeme eine nur unzureichende Spezifität für prä- und frühmaligne Läsionen gegenüber den nichtmalignen Gewebeveränderungen, was aus den Kurven für die spektrale Fluoreszenzintensität der Fig. 5 bis 10 der anliegenden Zeichnung hervorgeht: In weiten Bereichen des Anregungsbandes ergibt sich praktisch keine Unterscheidbarkeit zwischen metaplastischem und entzündetem Gewebe einerseits und dysplastischem Gewebe und Carcinoma in situ andererseits.

Der Erfindung liegt daher die Aufgabe zu Grunde, eine Vorrichtung zur bildgebenden Diagnose von Gewebe, insbesondere für die Untersuchung des Bronchialbereichs, unter wahlweiser Anwendung der diagnostischen Weißlicht-Endoskopie DWLE und der diagnostischen Auto-Fluoreszenz-Endoskopie DAFE zu schaffen, welches außerdem den eingangs aufgelisteten Forderungen gerecht wird.

Dabei stehen an erster Stelle eine hohe Sensitivität für die prä- und frühmalignen Gewebestellen gegenüber dem gesunden Gewebe bei gleichzeitig hoher Spezifität für prä- und frühmaligne Läsionen gegenüber den nichtmalignen Gewebeatypien. Das bedeutet konkret, dass sich diese Läsionen einerseits im Autofluoreszenzbild optisch deutlich vom gesunden Gewebe abheben sollen, dass sie sich aber andererseits auch von den nichtmalignen Gewebeatypien wie z. B. Entzündungen und Metaplasien klar unterscheiden lassen. Wichtig ist auch, dass dieses System ohne Einschränkungen tauglich ist für die DWLE. Ein Wechsel zwischen der DAFE und der DWLE soll schnell und auf einfachste Weise realisiert werden können. Das System soll auch handlich und leicht und außerdem preiswert sein.

Optional soll es mittels eines weiteren Arbeitsmodus DAFE II möglich sein, metaplastische Gewebeveränderungen nicht nur von den präund frühmalignen Läsionen, sondern auch von gesundem Gewebe und von Entzündungen unterscheiden zu können.

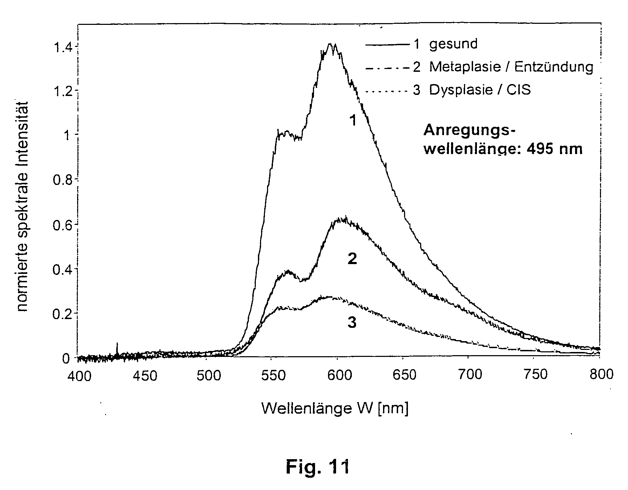

Grundlage für die Konzeption, Realisierung und Optimierung dieses Systems ist die systematische und detaillierte Untersuchung des Verhaltens der spektralen Fluoreszenzintensität menschlichen Gewebes, im hier betrachteten konkreten Fall der Bronchialschleimhaut, in Abhängigkeit vom Gewebezustand einerseits und in Abhängigkeit von der Fluoreszenz-Anregungswellenlänge andererseits. Die Fig. 1 bis 11 zeigen die spektralen Kurvenverläufe für diverse, äquidistante, eng beieinander liegende Anregungswellenlängen, und zwar jeweils für unterschiedliche Gewebezustände, nämlich für gesundes Gewebe, metaplastisches und entzündetes Gewebe sowie für Dysplasien und Carcinoma in situ.

Aus den Schaubildern wird deutlich, dass es zwar bereits bei Fluoreszenz-Anregungswellenlängen im UV einen Unterschied der spektralen Fluoreszenzintensitäten zwischen gesundem Gewebe und prä- und frühmalignem Gewebe gibt, nämlich in der Art, dass diese Intensitäten für gesundes Gewebe über den gesamten Emissionsbereich höher sind als für prä- und frühmalignes Gewebe, dass aber dieser Unterschied erst bei Anregungswellenlängen im Sichtbaren stark ausgeprägt ist. Dieser Unterschied ist jeweils am deutlichsten bei den Emissionsmaxima, also im Blauen und Grünen, und damit in dem Wellenlängenbereich, welcher im Wesentlichen für die Farbe und die Helligkeit des Gewebes und damit für das Erscheinungsbild des Gewebes ganz allgemein im Autofluoreszenzbild verantwortlich ist. Im Roten, also in dem Spektralbereich, wo die Fluoreszenz schwächer wird, weshalb dieser Spektralbereich weniger Einfluss nimmt auf die Erscheinungsform des Gewebes im Autofluoreszenzbild, nähern sich die Kurven einander an.

Quantitativ heißt das, dass bei Anregung mit Wellenlängen im UV (siehe Fig. 1 bis 3) das gesunde Gewebe im Bereich des Fluoreszenzmaximums nur zwei bis drei mal heller ist als prä- und frühmalignes Gewebe, während dieser Faktor bei Anregung des Gewebes mit Strahlung aus dem Sichtbaren im entsprechenden Emissionsbereich, also wiederum im Bereich des Fluoreszenzmaximums, bis auf zehn ansteigt (siehe die Fig. 5 bis 11).

Dementsprechend ist im Autofluoreszenzbild der Helligkeitsunterschied und damit die Sensitivität für prä- und frühmaligne Läsionen gegenüber gesundem Gewebe bei Fluoresezenz-Anregungswellenlängen im UV nicht optimal, hingegen bei Anregung im Sichtbaren deutlich besser.

Allerdings ist für die meisten Anregungswellenlängen, sowohl im UV als auch im Sichtbaren, die spektrale Fluoreszenzintensität für entzündetes und metaplastisches Gewebe gegenüber dem gesunden Gewebe ähnlich gering wie für Dysplasien und Carcinoma in situ, (siehe dazu vor allen Dingen die Fig. 2, 3, 4, 6, 7, 9 und 11). Dementsprechend erscheinen im Autofluoreszenzbild bei diesen Anregungswellenlängen die nichtmalignen Gewebeatypien gegenüber dem gesunden Gewebe ähnlich dunkel wie die prä- und frühmalignen Gewebeatypien. Eine Differenzierung zwischen nichtmalignen Gewebeatypien und prä- und frühmalignen Gewebeatypien ist im Autofluoreszenzbild damit praktisch nicht möglich.

Dies resultiert in einer schlechten Spezifität für prä- und frühmaligne Läsionen gegenüber den nichtmalignen Gewebeatypien bei diesen Anregungswellenlängen. Für die Praxis bedeutet dies, dass auch von den nichtmalignen atypischen Gewebestellen eine Biopsie gemacht werden muss, da diese im Autofluoreszenzbild gegenüber dem gesunden Gewebe ähnlich dunkel erscheinen wie die prä- und frühmalignen Läsionen und damit von diesen praktisch nicht zu unterscheiden sind; erst der Pathologe kann nach Untersuchung des bioptischen Materials Auskunft über den tatsächlichen Gewebezustand geben.

Diesbezüglich eine Ausnahme bilden die Anregungswellenlängen im Bereich von 405 nm und 450 nm, (siehe die Fig. 5 und 8). Insbesondere bei einer Anregung mit Licht im Bereich der Wellenlänge von 405 nm unterscheidet sich die spektrale Fluoreszenzintensität von nichtmalignen Gewebeatypien deutlich von derjenigen für die prä- und frühmalignen Gewebeatypien.

Quantitativ heißt das, dass auf der einen Seite metaplastisches und entzündetes Gewebe im Emissionsmaximum bei etwa 500 nm um ungefähr einen Faktor acht intensiver fluoresziert als dysplastisches Gewebe und Carcinoma in situ. Auf der anderen Seite ist die spektrale Fluoreszenzintensität der nichtmalignen Gewebeatypien nur etwa 20% geringer als diejenige für gesundes Gewebe und ist somit mit letzterer vergleichbar.

Für die Praxis bedeutet dies, dass sich im Autofluoreszenzbild bei einer Fluoreszenz-Anregung im Bereich von 405 nm die prä- und frühmalignen Läsionen sowohl vom gesunden Gewebe, aber eben auch von den nichtmalignen Gewebeatypien hervorragend abheben. Von letzteren braucht deshalb keine Biopsie gemacht zu werden, um vom Pathologen endgültige Klarheit darüber zu erhalten ob es sich um prä- und frühmalignes Gewebe handelt oder nicht. Nichtmaligne Gewebeatypien können praktisch nicht mehr mit prä- und frühmalignen Läsionen verwechselt werden.

Die Fluoreszenz-Anregung in einem Wellenlängenbereich um 405 nm resultiert somit in einer ausgezeichneten Sensitivität und Spezifität für prä- und frühmaligne Läsionen. Die nichtmalignen Gewebeatypien dagegen erscheinen im Autofluoreszenzbild praktisch wie gesundes Gewebe. Ein vergleichbares aber doch in dieser Art etwas weniger stark ausgeprägtes Verhalten findet man bei einer Fluoreszenz-Anregungswellenlänge im Bereich um 450 nm, (siehe Fig. 8).

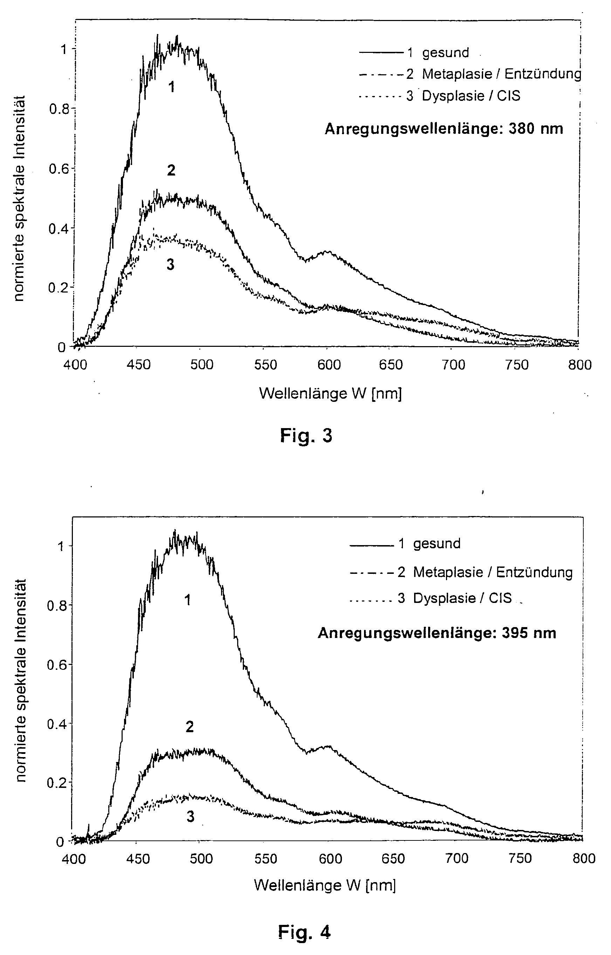

Eine zu starke Verbreiterung dieser beiden Wellenlängenbereiche für die Fluoreszenz-Anregung, um beispielsweise mit einer breitbandigen Lichtquelle mittels optischer Filtertechnik eine Steigerung der Helligkeit des Autofluoreszenzbildes zu erzeugen, führt jedoch zu einer Verschlechterung der Spezifität, da sich bei den benachbarten Anregungs-Wellenlängenbereichen entsprechend der Fig. 4 und 6 bzw. 7 und 9 die Kurven für die spektrale Fluoreszenzintensität für nichtmaligne und prä- und frühmaligne Gewebeatypien einander sehr ähnlich sind.

Eine die Aufgabe der Erfindung lösende Vorrichtung ist im Anspruch 1 angegeben. Vorteilhafte Weiterbildungen einer solchen Vorrichtung ergeben sich aus den Unteransprüchen.

Bei der erfindungsgemäßen Vorrichtung stellt die Lichtquelle in einer ersten Systemvariante im Arbeitsmodus der DAFE entweder ausschließlich oder zum größten Teil Licht aus einem schmalen Wellenlängenband um 405 nm bereit. Entsprechend den obigen Erläuterungen resultiert dies im Autofluoreszenzbild in einer hervorragenden Sensitivität und Spezifität für prä- und frühmaligne Läsionen.

Das System ist mittels Tastendruck, Sprachsteuerung und / oder Betätigung eines Fußschalters schnell und einfach umschaltbar zwischen der DAFE und der DWLE.

Um das vergleichsweise intensitätsschwache Autofluoreszenzbild sichtbar zu machen, ist im Bildpfad, also zwischen Gewebe und Detektoreinheit, ein optisches Blockfilter untergebracht, welches das gesamte oder zumindest den größten Anteil des von der Lichtquelle im DAFE-Modus applizierten und am Gewebe remittierten Lichtes blockt. Findet in dem System ein Videoendoskop Verwendung, ist aus technisch-konstruktiven Gründen dieses Blockfilter fest, also nicht ein- und ausschwenkbar, im Bildpfad montiert. Es befindet sich damit auch nach dem Umschalten in den Modus der DWLE im Strahlengang der Bildstrahlen. Deshalb soll dieses Blockfilter andererseits ein möglichst breites spektrales Band des Sichtbaren transmittieren, um im Modus der DWLE durch die Möglichkeit der Detektion nahezu aller spektralen Bereiche des Sichtbaren ein optimales Farbbild mit einer hervorragenden farblichen Gewebedifferenzierung zu ermöglichen.

Auch in dieser Hinsicht ist das Fluoreszenz-Anregungsband um 405 nm von Vorteil, da sich dieses am kurzwelligen Rand des Sichtbaren befindet, nämlich dort, wo die Empfindlichkeit des menschlichen Auges ohnehin noch fast vernachlässigbar ist. Wird das optische Blockfilter nun so spezifiziert, dass dessen Transmissionsband sich unmittelbar dem Transmissionsband des Anregungsfilters um 405 nm im Sichtbaren anschließt und über den gesamten sichtbaren spektralen Bereich erstreckt, dann ist die geforderte, praktisch uneingeschränkte Farbwiedergabe nach dem Umschalten in den Modus der DWLE gewährleistet.

Wie bereits oben bemerkt und wie aus Fig. 5 ersichtlich wird, sind die Kurven für die spektrale Fluoreszenzintensität von gesundem Gewebe und metaplastischem und entzündetem Gewebe gegenüber der Kurve für die spektrale Fluoreszenzintensität von Dysplasien und Carcinoma in situ bei Anregung mit Wellenlängen um 405 nm einander sehr ähnlich. Dies bedeutet für die Praxis und den Anwender, dass im Autofluoreszenzbild metaplastisches und entzündetes Gewebe von gesundem Gewebe praktisch nicht zu unterscheiden ist: Beide Gewebeformen erscheinen gegenüber den prä- und frühmalignen Formen nahezu gleich hell. Für bestimmte Anwendungen kann es jedoch von Interesse sein, insbesondere metaplastisches Gewebe von gesundem Gewebe differenzieren zu können. So wird beispielsweise darüber nachgedacht, Patienten mit Metaplasien chemopräventiv zu behandeln.

In einer zweiten Systemvariante, welche eine Erweiterung der oben beschriebenen ersten Systemvariante mit nur einem Autofluoreszenzmodus für eine erste Form der diagnostischen Autofluoreszenz-Endoskopie, DAFE bzw. DAFE darstellt, wird deshalb ein zweites Anregungsband für eine zweite Form der diagnostischen Auto-Fluoreszenz-Endoskopie, DAFE II, bereitgestellt für einen zusätzlichen zweiten Autofluoreszenzmodus.

Um den oben erläuterten Vorteil der praktisch uneingeschränkten Farbwiedergabe bei der DWLE zu erhalten, und zwar trotz des evt. im Bildpfad unbeweglich montierten optischen Blockfilters, wird dieses zweite Anregungsband in den Wellenlängenbereich um 395 nm gelagert. So kann die spektrale Breite des Transmissionsbandes des optischen Blockfilters im Sichtbaren erhalten bleiben und es können nahezu alle spektralen Komponenten des Sichtbaren von der Detektoreinheit erfasst werden, was Voraussetzung für ein optimales Weißlichtbild ist.

Andererseits ist bei einer Anregung um 395 nm (siehe Fig. 4) die spektrale Fluoreszenzintensität von metaplastischem und entzündetem Gewebe gegenüber der Intensität von gesundem Gewebe fast identisch mit der spektralen Fluoreszenzintensität von prä- und frühmalignem Gewebe. Das bedeutet für die Praxis und den Anwender, dass sich im Autofluoreszenzbild metaplastisches und entzündetes Gewebe einerseits und Dysplasien und Carcinoma in situ andererseits durch ihre reduzierte Helligkeit deutlich vom gesunden Gewebe abheben, dass aber alle diese Gewebeatypien vergleichbar dunkel erscheinen und insofern untereinander fast nicht zu unterscheiden sind.

Diesbezüglich, und das heißt auch im Sinne einer guten Sensitivität bei gleichzeitig guter Spezifität in der oben beschriebenen Form, ist die kombinierte bzw. sequenzielle Applikation von Strahlung zweier unterschiedlicher, genau definierter Fluoreszenzanregungsbänder in zwei unterschiedlichen Auto-Fluoreszenz-Arbeitsmoden, nämlich der DAFE bzw. DAFE I und der DAFE II, von Vorteil: Mit der DAFE II, also mit der Fluoreszenz-Anregung in einem Wellenlängenband um 395 nm, werden sämtliche Formen von Gewebeatypien, also Metaplasien, Entzündungen, Dysplasien, Carcinoma in situ, usw. aufgesucht und lokalisiert. Alle diese Gewebeatypien heben sich im Autofluoreszenzbild bei der DAFE II durch ihre geringe Fluoreszenzintensität und damit durch ihr dunkles Erscheinungsbild vom umgebenden, hell wirkenden gesunden Gewebe ab. Wird anschließend in den anderen Arbeitsmodus, die DAFE I, gewechselt, dann wirken im Autofluoreszenzbild die nichtmalignen Gewebeatypien praktisch genauso hell wie das gesunde Gewebe, während die prä- und frühmalignen Läsionen weiterhin dunkel erscheinen.

Die prä- und frühmalignen Gewebeatypien erscheinen also in beiden Autofluoreszenzmoden dunkel, während die nichtmalignen Gewebeatypien beim Wechsel von der DAFE II in die DAFE I von dunkel zu hell wechseln. Um schließlich noch die Metaplasien von den Entzündungen differenzieren zu können, muss in den Arbeitsmodus der DWLE gewechselt werden: Während dort die Metaplasien von gesundem Gewebe praktisch nicht unterschieden werden können, erscheinen die Entzündungen gerötet.

Wenn eine minimale farbliche Beeinträchtigung des Weißlichtbildes akzeptiert werden kann, dann kann das Anregungsband bei der DAFE II auch in das Sichtbare, in direkten Anschluss an das Anregungsband bei der DAFE I, und damit in den Bereich um 420 nm gelagert werden. Entsprechend dieser Verlagerung des Anregungsbandes muss aber dann auch der Transmissionsbereich des optischen Blockfilters im Bildpfad modifiziert werden: Nun bildet die langwellige spektrale Kante des Anregungsfilters der DAFE II die Grenze für die ansteigende spektrale Kante des optischen Blockfilters, und entsprechend wird der Transmissionsbereich dieses Blockfilters im Sichtbaren eingeengt.

Weitere vorteilhafte Merkmale der erfindungsgemäßen Vorrichtung ergeben sich aus der Beschreibung von in der Zeichnung schematisch dargestellten Ausführungsbeispielen. In der Zeichnung zeigen:
- Fig. 1 bis 11: die typischen spektralen Fluoreszenzintensitäten von menschlichem Bronchialgewebe für unterschiedlichen Gewebezustände bei unterschiedlichen Anregungswellenlängen, normiert auf das Fluoreszenzmaximum von gesundem Gewebe,
- Fig. 12: schematisch den Aufbau einer Diagnosevorrichtung nach der Erfindung,
- Fig. 13: eine erste mögliche Ausführungsform der Optikeinheit der Lichtquelle,
- Fig. 14: schematisch eine mögliche Ausführungsform des spektralen Transmissionverhaltens T des Fluoreszenz-Anregungsfilters bei der DAFE I über der Wellenlänge W in Nanometer nm,
- Fig. 15: schematisch eine mögliche Ausführungsform des spektralen Transmissionverhaltens T des auf das Fluoreszenz Anregungsfilter der Fig. 14 abgestimmten Blockfilters über der Wellenlänge W in Nanometer nm,
- Fig. 16: die spektrale Hellempfindlichkeit V des menschlichen Auges über der Wellenlänge W in Nanometer nm,
- Fig. 17: schematisch eine erste mögliche Ausführungsform der spektralen Transmissionverhaltens T des Fluoreszenz-Anregungsfilters bei der DAFE II über der Wellenlänge W in Nanometer nm,
- Fig. 18: schematisch eine zweite mögliche Ausführungsform des spektralen Transmisssionverhaltens T des Floureszenz-Anregungsfilters bei der DAFE II über der Wellenlänge W in Nanometer nm,
- Fig. 19: schematisch eine mögliche Ausführungsform des spektralen Transmissionverhaltens T des auf das Fluoreszenz-Anregungsfilter bei der DAFE II der Fig. 18 abgestimmten Blockfilters über der Wellenlänge W in Nanometer nm,
- Fig. 20: eine zweite mögliche Ausführungsform der Optikeinheit der Lichtquelle und
- Fig. 21: die zweite mögliche Ausführungsform der Optikeinheit der Lichtquelle der Fig. 20 mit optionaler Verfügbarkeit eines zweiten Autofluoreszenzmodus " DAFE II

Die Fig. 1 bis 11 zeigen die typischen spektralen Fluoreszenzintensitäten von menschlichem Bronchialgewebe für unterschiedliche Gewebezustände bei unterschiedlichen Anregungswellenlängen. Auf diese Schaubilder wurde bereits oben im Detail eingegangen.

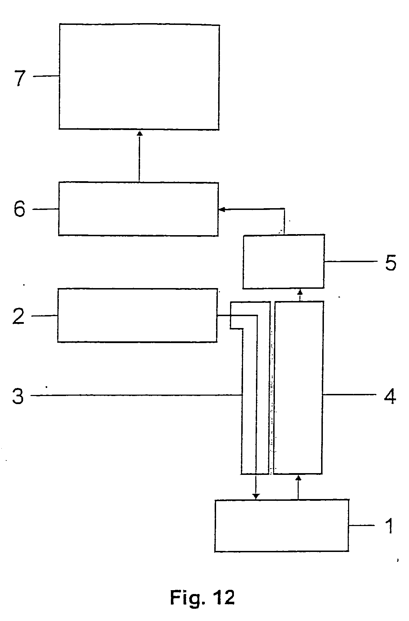

Fig. 12 zeigt schematisch, nämlich in Form eines Blockbildes, den Aufbau der Vorrichtung für die kombinierte diagnostische Weißlicht-Endoskopie DWLE, die diagnostische Auto-Fluoreszenz-Endoskopie DAFE bzw. DAFE I und optional die diagnostische Auto-Fluoreszenz-Endoskopie DAFE II.

Die Vorrichtung zur bildgebenden Diagnose des Gewebes 1 besteht aus einer Lichtquelle 2, welche im Arbeitsmodus der DWLE Weißlicht für die Beleuchtung und in den Arbeitsmoden der DAFE I und der DAFE II unterschiedliche Strahlung für die jeweilige Form der Fluoreszenz-Anregung über einen Lichtleiter 3 an das Gewebe 1 abgibt. Der Lichtleiter 3 kann aus einer Einzelfaser, einem Faserbündel oder einem Flüssiglichtleiter oder auch aus einer Kombination dieser Elemente bestehen.

Sowohl das bei der DWLE durch remittierte Strahlung erzeugte Bild als auch die bei der DAFE I und DAFE II jeweils erzeugten Fluoreszenzbilder werden über eine Bildübertragungseinheit 4 einer Bildaufnahmeeinheit 5 zugeführt, wo eine Umwandlung der optischen Signale in elektrische Signale stattfindet. Letztere werden an eine Bildverarbeitungseinheit 6 weitergeleitet, wo eine Aufbereitung der elektrischen Signale stattfindet, um beispielsweise ein Bild auf einem Monitor 7 zu erzeugen. Gleichermaßen ist aber auch die Aufzeichnung mit einem Videorecorder oder einer anderen videotechnischen Einrichtung möglich. Denkbar ist auch die endoskopische Direktbeobachtung, bei welcher die Bildaufnahmeeinheit 5 die Bildverarbeitungseinheit 6 und der Monitor 7 durch das Auge des menschlichen Betrachters ersetzt werden. Im Bereich der Bildübertragungseinheit 4 ist das optische Blockfilter untergebracht. Dieses ist in der Fig. 12 nicht eingezeichnet.

Wird für die Bilddarstellung ein Videoendoskop, also ein sog. Chipendoskop, verwendet, bei welchem der Videochip im Endoskop selbst untergebracht ist, dann handelt es sich bei der Bildübertragungseinheit 4 im Wesentlichen um ein Objektiv und bei der Bildaufnahmeeinheit 5 um das Sensorsystem des Videoendoskops. Die Bildverarbeitungseinheit 6 stellt den zugehörigen Controller dar. Dabei handelt es sich sowohl beim Chipendoskop als auch beim Controller um Komponenten, die uneingeschränkt für die DWLE verwendet werden können.

Findet hingegen eine Kamera Verwendung, dann besteht die Bildübertragungseinheit 4 aus einem Endoskop-Objektiv, einem sich daran anschließenden Bildfaserbündel oder einem Linsen- oder Stablinsensystem, welche beide Teil eines Endoskops sind, aus einem Endoskop-Okular und evt. einem Kamera-Objektiv. Diese Komponenten übertragen das Bild vom Gewebe 1 auf die Bildaufnahmeeinheit 5. Letztere wird gebildet vom Sensorsystem eines Kamerakopfes. Bei der Bildverarbeitungseinheit 6 handelt es sich um den Kameratreiber. Auch bei der Kamera handelt es sich um ein Gerät, welches uneingeschränkt für die DWLE verwendet werden kann. Es kann sich sogar um eine konventionelle medizinische Kamera handeln.

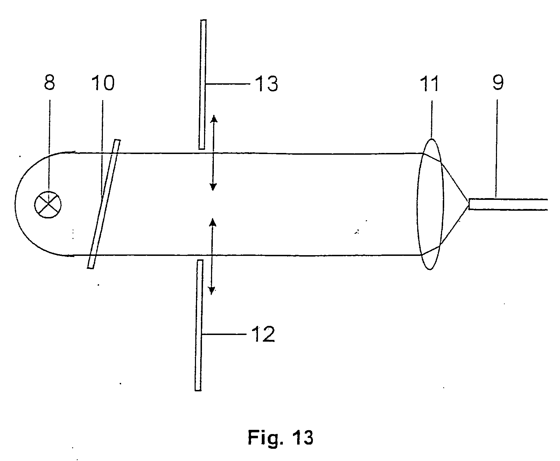

Fig. 13 zeigt eine erste mögliche Ausführungsform der Optikeinheit der Lichtquelle 2 aus Fig. 12. Diese erste mögliche Ausführungsform ist dadurch gekennzeichnet, dass ein einziges Leuchtmittel 8 Verwendung findet. Bei diesem Leuchtmittel 8 handelt es sich um eine inkohärente, breitbandig im sichtbaren Spektralbereich emittierende Lampe. Wird bei dem idealerweise weißes Licht emittierenden Leuchtmittel eine Kurzbogenlampe verwendet, beispielsweise eine Xenonlampe, eine Mischgaslampe mit entsprechender Gasmischung oder eine Lampe mit Quecksilberanteilen, gelingt die Strahlungseinkopplung in den Lichtleiter 9, welcher dem Lichtleiter 3 in Fig. 12 entspricht, besonders gut. Alternativ kann als Leuchtmittel auch eine Halogenlampe verwendet werden.

Im Ausführungsbeispiel der Fig. 13 findet eine Parabolspiegellampe Verwendung, welche ein paralleles Strahlenbündel abgibt. Das sich fest im Strahlengang befindliche Filter 10 blockiert die IR- und weitgehend die UV-Strahlungsanteile des Leuchtmittels 8. Im Brennpunkt der Linse 11 befindet sich der Lichtleiter 9, in welchen die gefilterte Strahlung des Leuchtmittels 8 eingekoppelt wird.

Beim Umschalten in den Arbeitsmodus der DAFE bzw. DAFE I wird das Fluoreszenz-Anregungsfilter 12 in den parallelen Strahlengang des Leuchtmittels 8 eingeschwenkt. Dies geschieht beispielsweise über das Betätigen eines Fußschalters oder über einen Tastendruck an der Bedienfront der Lichtquelle oder über Sprachsteuerung. Diese Elemente sind mit einer Steuereinheit verbunden, die den Filterschwenkmechanismus kontrolliert und die in der Lichtquelle untergebracht sein kann. Diese den Schwenkvorgang von Filter 12 betreffenden Komponenten sind in Fig. 13 nicht abgebildet. Beim Umschalten in den Arbeitsmodus der DWLE wird das Fluoreszenz-Anregungsfilter 12 aus dem Strahlengang ausgeschwenkt. Dies geschieht in der gleichen Weise wie das Einschwenken.

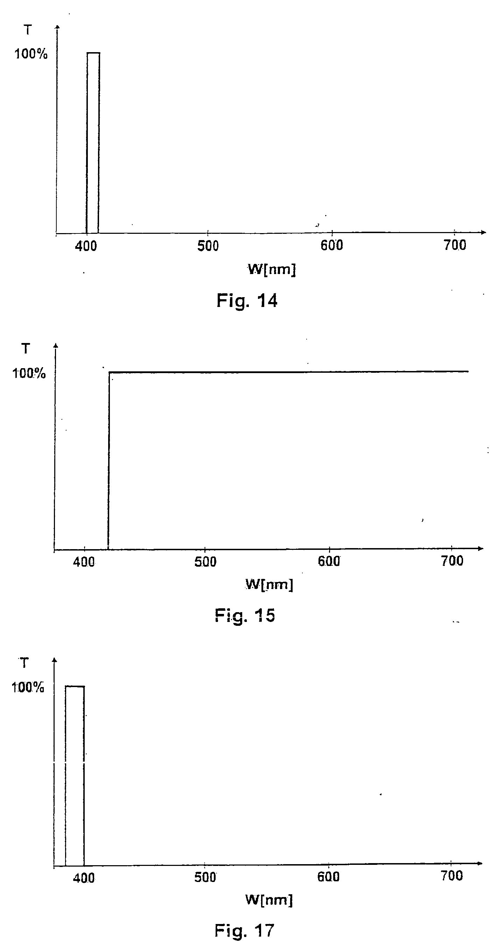

Fig. 14 zeigt schematisch eine mögliche Ausführungsform des spektralen Transmissionsverhaltens T des Fluoreszenz-Anregungsfilters 12 bei der DAFE bzw. DAFE I über der Wellenlänge W in Nanometer nm. Gemäß den obigen Erläuterungen hinsichtlich einer guten Sensitivität für präund frühmaligne Läsionen gegenüber gesundem Gewebe in Kombination mit einer guten Spezifität für prä- und frühmaligne Läsionen gegenüber nichtmalignen Gewebeatypien wird eine Fluoreszenz-Anregung mit Strahlung aus einem schmalen spektralen Bereich, welcher die Wellenlänge 405 nm einschließt, vorgenommen. Das Filter sollte jedoch zumindest so spezifiziert sein, dass der größte Anteil der Strahlung für die Fluoreszenz-Anregung einem Wellenlängenband entstammt, dessen Breite nicht größer als 20 nm ist und zumindest eine der Wellenlängen 405 nm ± 5 nm einschließt. Für eine gute Anregung sollte das Fluoreszenz-Anregungsfilter 12 in seinem Transmissionsband eine Durchlässigkeit von mindestens 50% haben. Idealerweise sollte jedoch die Transmission gegen 100% gehen, um so durch eine intensitätsstarke Fluoreszenz-Anregung ohne zusätzliche bildhelligkeitssteigernde Mittel wie Bildverstärker o. ä. ein genügend helles Autofluoreszenzbild zu erzielen. Außerhalb dieses schmalen Transmissionsbandes um 405 nm liegt die Durchlässigkeit nahezu überall im Bereich von 0%.

Soll jedoch im Autofluoreszenzbild zusätzlich eine Farbreferenz etabliert werden, was dadurch realisiert wird, dass dem Fluoreszenzbild zusätzlich ein Bild aus am Gewebe remittierter Strahlung überlagert wird, wie dies bereits an anderen Stellen beschrieben wurde, dann ist die Transmission des Fluoreszenz-Anregungsfilters in dem entsprechenden Wellenlängenbereich oder in den entsprechenden Wellenlängenbereichen, dem oder denen die detektierte, am Gewebe remittierte Strahlung entstammen soll, gleichfalls von Null verschieden.

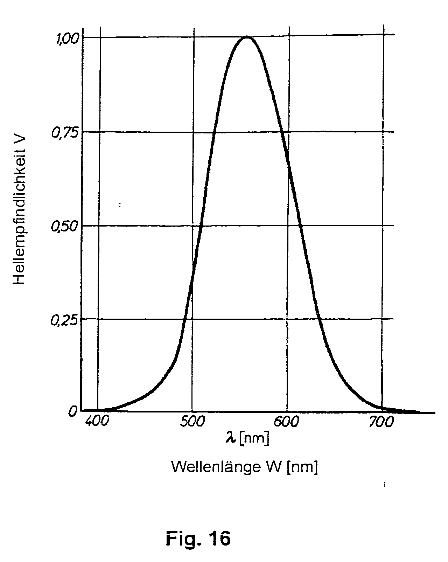

Fig. 15 zeigt schematisch eine mögliche Ausführungsform des spektralen Transmissionsverhaltens T des auf das Fluoreszenz-Anregungsfilter der Fig. 14 abgestimmten optischen Blockfilters, welches sich im Bildpfad des Systems, und zwar vor der Detektoreinheit, befindet, über der Wellenlänge W in Nanometer nm. Entsprechend den obigen Ausführungen orientiert sich die spektrale Transmissionscharakteristik des Filters u. a. an derjenigen des Filters 12, dessen Transmissionsverlauf in Fig. 14 schematisch dargestellt ist: Im Bereich des Transmissionsbandes des Fluoreszenz-Anregungsfilters 12 um 405 nm geht die Transmission des Blockfilters gegen Null. Jedoch in dem sich unmittelbar daran anschließenden längerwelligen, sichtbaren Spektralbereich liegt die Transmission ungefähr gleichmäßig bei über 50%, idealerweise geht sie gegen 100%. Das bedeutet, dass die ansteigende Filterkante des Blockfilters bereits im Bereich von 420 nm liegen kann und damit in einem Wellenlängenbereich, in welchem die spektrale Hellempfindlichkeit V des menschlichen Auges immer noch äußerst gering ist, wie es aus der Fig. 16, in welcher dieser Kurvenverlauf festgehalten ist, ersichtlich wird. Das wiederum heißt, dass bei dieser Vorgehensweise nach Umschalten in den Modus der DWLE auch bei aus dem Bildpfad nicht-ausschwenkbarem Blockfilter praktisch alle für das menschliche Farbempfinden relevanten spektralen Wellenlängenbereiche detektiert werden können. Dies resultiert in einer optimalen Farbwiedergabe der erfindungsgemäßen Vorrichtung.

Entsprechend den obigen Erläuterungen besteht in einer zweiten Systemvariante der erfindungsgemäßen Vorrichtung optional die Möglichkeit, das Gewebe mit Strahlung aus einem zweiten spektralen Band anzuregen, um in einem zweiten Autofluoreszenzmodus, DAFE II, eine Differenzierung zwischen nichtmalignen Gewebeatypien und gesundem Gewebe zu ermöglichen und schließlich entsprechend den obigen Ausführungen in Kombination mit den beiden anderen Arbeitsmoden metaplastisches Gewebe lokalisieren zu können. Bei der in der Fig. 13 dargestellten ersten Ausführungsform der Optikeinheit der Lichtquelle 2, welche durch die Beschränkung auf ein einziges Leuchtmittels 8 charakterisiert ist, wird dies durch die Möglichkeit des Einschwenkens eines zweiten optischen Filters 13 in den Strahlengang des Leuchtmittels 8 realisiert.

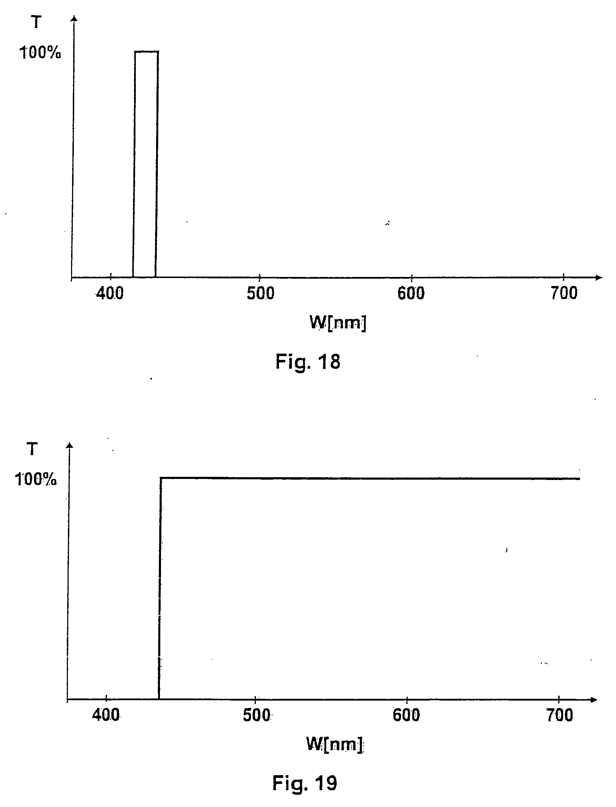

Fig. 17 zeigt schematisch eine mögliche Ausführungsform des spektralen Transmissionsverhaltens T des Fluoreszenz-Anregungsfilters bei der DAFE II über der Wellenlänge W in Nanometer nm. Entsprechend dieser schematischen Darstellung und gemäß den obigen Erläuterungen beschränkt sich das Transmissionsband für die Fluoreszenz-Anregung dieses optischen Bandpassfilters idealerweise auf einen engen Bereich um eine Wellenlänge von 395 nm. Zumindest aber sollte der größte Anteil der Strahlung für die Fluoreszenz-Anregung einem Bereich von weniger als 30 nm Breite, welcher mindestens eine der Wellenlängen von 395 nm ± 5 nm einschließt, entstammen, und der größte Strahlungsanteil sollte durch eine Wellenlänge von weniger als 400 nm charakterisiert sein.

Dadurch wird einerseits gemäß der Fig. 3 eine gute Differenzierung zwischen nichtmalignen Gewebeatypien und gesundem Gewebe ermöglicht, andererseits kann aber auch die oben beschriebene und in Fig. 15 schematisch dargestellte, hinsichtlich der Fargbwiedergabe bei der DWLE günstige Transmissionscharakteristik des optischen Blockfilters im Bildpfad beibehalten werden.

Gemäß den Fig. 6 und 7 kann sich das Transmissionsband für das optische Filter bei der DAFE II im Hinblick auf eine gute Differenzierung zwischen gesundem Gewebe und nichtmalignem atypischem Gewebe auch im Sichtbaren an jenes bei der DAFE I anschließen. Allerdings muss dann auch die Transmissionscharakteristik des Blockfilters geändert werden, und zwar derart, dass die spektrale Kante ins Langwellige verschoben wird, so dass es praktisch keine Überlappung der Transmissionsbänder des Blockfilters und des Filters für die DAFE II gibt. Um die Farbwiedergabe bei der DWLE nicht deutlich zu beeinträchtigen, sollte die spektrale Kante des Blockfilters noch immer in einem Bereich liegen, in welchem die spektrale Hellempfindlichkeit V des menschlichen Auges vergleichsweise gering ist, also beispielsweise höchstens bei 475 nm, idealerweise aber bei einer Wellenlänge von geringerem Wert.

Das Transmissionsband des Fluoreszenz-Anregungsfilters für die DAFE II sollte so spezifiziert sein, dass der größte Teil der Strahlung für die Anregung aus einem Wellenlängenband mit einer Breite von weniger als 35 nm entstammt, und dessen langwellige Kante bei einer Wellenlänge von höchstens 445 nm liegt. Eine stärkere Einengung des Transmissionsbereiches für die DAFE II um 420 nm ist jedoch hinsichtlich der Gewebedifferenzierung und im Hinblick auf die Optimierungsanstrengungen der Farbwiedergabe bei der DWLE von Vorteil. Dann nämlich kann auch die spektrale Kante des Blockfilters im Bildpfad entsprechend an den kurzwelligen Rand des Sichtbaren verschoben werden.

Fig. 18 zeigt schematisch eine zweite mögliche Ausführungsform des spektralen Transmissionverhaltens T des Fluoreszenz-Anregungsfilters bei der DAFE II über der Wellenlänge W in Nanometern nm, welche dadurch gekennzeichnet ist, dass sich das Transmissionsband auf einen schmalen Bereich um 420 nm beschränkt.

Fig. 19 zeigt schematisch eine mögliche Ausführungsform des spektralen Transmissionverhaltens T einer modifizierten Form des Blockfilters der Fig. 14, welches nun auf die zweite mögliche Ausführungsform des Fluoreszenz-Anregungsfilters bei der DAFE II, dessen Transmissionscharakteristik in Fig. 18 dargestellt ist, abgestimmt ist. Diese Ausführungsform zeichnet sich dadurch aus, dass sich der Transmissionsbereich unmittelbar an jenen schmalen Transmissionsbereich des durch die Fig. 18 charakterisierten Filters anschließt, dass also die spektrale Kante im Bereich von etwa 435 nm liegt.

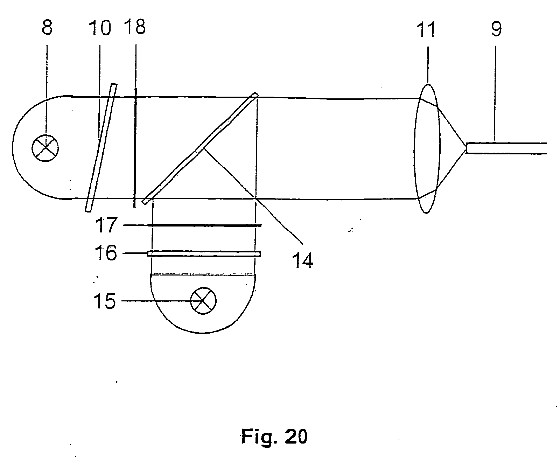

Fig. 20 zeigt eine zweite mögliche Ausführungsform der Optikeinheit der Lichtquelle 2, welche dadurch charakterisiert ist, dass die Lichtquelle 2 aus zwei Leuchtmitteln besteht. Der Grundaufbau dieser zweiten möglichen Ausführungsform der Optikeinheit, bestehend aus Leuchtmittel 8, Lichtleiter 9, Filter 10 und Linse 11, ist identisch mit dem Aufbau der Fig. 13 und wird daher an dieser Stelle nicht nochmals beschrieben. Die Strahlung für die Fluoreszenz-Anregung bei der DAFE bzw. DAFE I wird jedoch in dieser Ausführungsform nicht durch die optische Filterung der vom breitbandigen Leuchtmittel 8 emittierten Strahlung realisiert.

Anstelle des in den Strahlengang des Leuchtmittels 8 ein- und ausschwenkbaren Filters 12 (Fig. 13) ist nun ein beschichtetes Glasplättchen 14 im Winkel von 45° gegenüber der optischen Achse des Leuchtmittels 8 in den Strahlengang eingebracht. Die Beschichtung ist dadurch charakterisiert, dass sie Strahlung mit einer Wellenlänge kleiner als 415 nm / ± 5 nm, also auch noch einen kleinen Anteil sichtbarer Strahlung, welche unter einem Winkel von 45° gegen die Oberflächennormale des Glasplättchens auftrifft, reflektiert und Strahlung mit einer Wellenlänge größer als 415 nm / ± 5 nm, welche unter einem Winkel von 45° gegen die Oberflächennormale des Glasplättchens auftrifft, transmittiert. Bei dem Leuchtmittel 15 der Fig. 20, welches so angeordnet ist, dass dessen kollimiertes Strahlenbündel in einem Winkel von 90° auf das kollimierte Strahlenbündel des Leuchtmittels 8 trifft, handelt es sich um eine Strahlungsquelle, die im Gegensatz zum breitbandigen Leuchtmittel 8 einen relativ hohen Strahlungsanteil im Bereich von 405 nm emittiert, beispielsweise um eine Gasentladungslampe mit einer entsprechend hohen Konzentration an Quecksilber, welches eine starke Emission u. a. bei 405 nm und eine etwas geringere bei 408 nm verursacht, einer Leuchtdiode oder einem Array von Leuchtdioden, welche im Bereich von 405 nm emittieren, oder einem Laser oder einer Laserdiode bzw. einem Array von Laserdioden, welche im Bereich von 405 nm emittieren.

Der Vorteil dieser zweiten möglichen Ausführungsform der Optikeinheit der Fig. 20 gegenüber der ersten möglichen Ausführungsform der Optikeinheit der Fig. 13 liegt in der Verfügbarkeit einer vergleichsweise hohen Strahlungsintensität in einem schmalen spektralen Bereich um 405 nm bei vergleichsweise niedriger Leistungsaufnahme und Wärmeerzeugung des Leuchtmittels durch den Einsatz von Strahlungsquellen, die gerade in diesem spektralen Bereich eine gewisse Form der Konzentration der Strahlungsemission haben, gegenüber einer breitbandigen Weißlichtquelle, wie sie z. B. beim Leuchtmittel 8 eingesetzt wird. Dies kann von großer Bedeutung sein, wenn es darum geht, ohne zusätzliche helligkeitssteigernde Mittel ausreichend helle Autofluoreszenzbilder zu erzeugen.

Abhängig von der Ausführungsform des Leuchtmittels 15 befindet sich im zweiten Strahlengang der Lichtquelle 2 ein weiteres optisches Bandpassfilter 16, dessen Transmissionsbereich im wesentlichen auf den spektralen Bereich um 405 nm eingeengt ist. Wird beispielsweise eine Gasentladungslampe mit Quecksilberanteilen als zweites Leuchtmittel 15 eingesetzt, wird durch dieses Filter 16 gewährleistet, dass ausschließlich die Emissionslinien bei 405 nm und 408 nm für die Fluoreszenz-Anregung verwendet werden, also ausschließlich jener Wellenlängenbereich zur Erzeugung des Autofluoreszenzbildes verwendet wird, bei welchem eine hohe Sensitivität für prä- und frühmaligen Läsionen gegenüber gesundem Gewebe in Kombination mit einer hohen Spezifität für prä- und frühmaligen Läsionen gegenüber den nichtmalignen Gewebeatypien erzielt wird.

Der Umschaltvorgang zwischen der DWLE und der DAFE bzw. DAFE I funktioniert ähnlich wie im Aufbau der Fig. 13 bereits erläutert über eine in der Fig. 20 nicht eingezeichnete zentrale Steuereinheit, welche über einen Fußschalter, über eine Taste an der Lichtquelle oder über Sprachsteuerung bedient wird. Diese zentrale Steuereinheit sorgt dafür, dass im Modus der DAFE bzw. DAFE I ein Shutter 17 aus dem Strahlengang des Leuchtmittels 15 aus- und ein Shutter 18 in den Strahlengang des Leuchtmittels 8 eingeschwenkt ist, so dass eine Bestrahlung des Gewebes mit Licht des Leuchtmittels 8 in diesem Modus nicht möglich ist. Umgekehrt sorgt diese zentrale Steuereinheit dafür, dass im Modus der DWLE der Shutter 18 aus dem Strahlengang des Leuchtmittels 8 ausund der Shutter 17 in den Strahlengang des Leuchtmittels 15 eingeschwenkt ist, so dass eine Bestrahlung des Gewebes mit Licht des Leuchtmittels 15 in diesem Modus nicht möglich ist.

Auch bei dieser zweiten möglichen Ausführungsform der Optikeinheit, welche dadurch gekennzeichnet ist, dass für den Modus der DAFE bzw. DAFE I ein zweites Leuchtmittel 15 verwendet wird, besteht in einer zweiten Variante der erfindungsgemäßen Vorrichtung optional die Möglichkeit, das Gewebe mit Strahlung aus einem zweiten spektralen Band anzuregen, um in einem zweiten Autofluoreszenzmodus DAFE II eine Differenzierung von nichtmalignen Gewebeatypien von gesundem Gewebe zu ermöglichen und schließlich entsprechend den obigen Ausführungen metaplastisches Gewebe lokalisieren zu können. Eine mögliche Form der Realisierung dieser zweiten Systemvariante und im Speziellen dieses zweiten Autofluoreszenzmodus geschieht über die optische Filterung der breitbandig durch das Leuchtmittel 8 bereitgestellten Strahlung, also prinzipiell vergleichbar mit der Vorgehensweise in der ersten Ausführungsform. Um im Hinblick auf Sensitivität und Spezifität vergleichbar gute Ergebnisse zu erzielen wie bei der ersten Ausführungsform, sind die spektralen Bandkanten des Filtersystems für die DAFE II in der zweiten Ausführungsform so zu wählen wie in der ersten Ausführungsform.

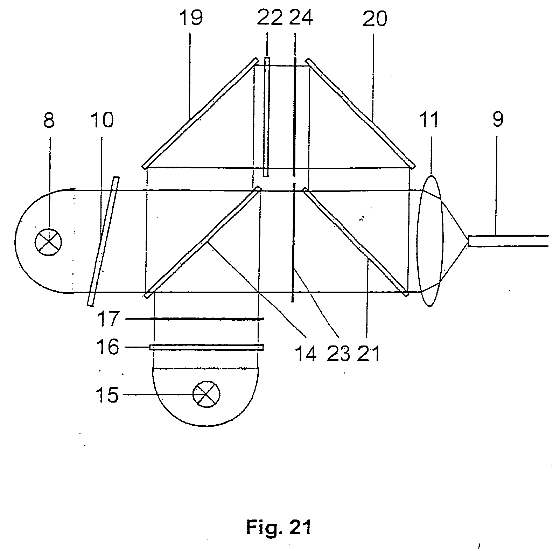

Die Fig. 21 zeigt schematisch den Aufbau. Dieser ist weitgehend identisch mit dem Aufbau gemäß Fig. 20. Abweichend von letzterem sind zwei weitere Spiegel 19 und 20 derart positioniert, dass die am Filter 14 reflektierte, kurzwellige Strahlung des Leuchtmittels 8 in senkrechter Richtung auf dessen optische Achse zurückgelenkt wird. Bei dem Element 21 handelt es sich um ein beschichtetes Glasplättchen. Die Beschichtung ist so spezifiziert, dass beispielsweise Strahlung mit einer Wellenlänge kleiner als 400 nm reflektiert wird. Damit ist gewährleistet, dass die Strahlung für die Fluoreszenz-Anregung bei der DAFE bzw. DAFE I aus dem schmalbandigen Bereich um 405 nm, welche vom Leuchtmittel 15 stammt, transmittiert wird, während die Strahlung für die Fluoreszenz-Anregung bei der DAFE II aus dem schmalbandigen Bereich um 395 nm durch Reflexion am Filter 21 zurück auf die optische Achse des Leuchtmittels 8 gelangt. Durch die Spezifikation des optischen Filters 22 ist der Bereich, welchem die Strahlung für die DAFE II entstammt, festgelegt. Durch entsprechende Betätigung der Shutter 17, 23 und 24 über eine nichteingezeichnete zentrale Steuereinheit beim Umschalten zwischen den diversen Moden wird dafür gesorgt, dass immer nur die jeweils erforderliche Strahlung an das Gewebe gelangt.

In einer weiteren denkbaren Ausführungsform der Optikeinheit der Lichtquelle 2 kann die Strahlung für die DAFE II auch durch ein drittes Leuchtmittel erzeugt und bereitgestellt werden.

Damit ist es gelungen, eine Vorrichtung zur bild gebenden Diagnose von Gewebe zu realisieren, welches allen den eingangs genannten Forderungen, insbesondere aber jenen nach hoher Sensitivität von präund frühmalignem Gewebe gegenüber gesundem Gewebe und hoher Spezifität von prä- und frühmalignem Gewebe gegenüber nichtmalignen Gewebeatypien gerecht wird.

## Patentansprüche

1. Vorrichtung zur bildgebenden Diagnose von Gewebe (1) unter wahlweiser Anwendung von mindestens zwei Diagnosemethoden, nämlich einem Arbeitsmodus zur diagnostischen Weißlicht-Endoskopie DWLE und einem ersten Arbeitsmodus zur diagnostischen Autofluoreszenz-Endoskopie DAFE bzw. DAFE I und optional einer dritten Diagnosemethode, nämlich einem zweiten Arbeitsmodus zur diagnostischen Autofluoreszenz-Endoskopie DAFE II, mit einer Lichtquelle (2), deren Licht über einen Lichtleiter (3) zum Gewebe (1) geleitet wird, und mit einer Bildübertragungseinheit (4), die entweder mit dem menschlichen Auge in Verbindung steht oder alternativ mit einer Bildaufnahmeeinheit (5), welche mit einer Bildverarbeitungseinheit (6) verbunden ist, über welche ein Monitor (7) mit einem Bildsignal versorgt wird, **dadurch gekennzeichnet, dass** der größte Teil der im Arbeitsmodus der DAFE bzw. DAFE I für die Fluoreszenz-Anregung bereitgestellten Strahlung einem schmalen spektralen Band entstammt, welches im Bereich der Wellenlänge von 405 nm liegt.

2. Vorrichtung nach Anspruch 1, **dadurch gekennzeichnet, dass** die Breite des schmalen spektralen Bandes, welchem der größte Teil der Fluoreszenz-Anregungsstrahlung für die DAFE bzw. DAFE I entstammt, nicht größer als 20 nm ist und dieses Band mindestens eine der Wellenlängen aus dem Bereich von 405 nm ± 5 nm einschließt.

3. Vorrichtung nach einem der Ansprüche 1 und 2, **dadurch gekennzeichnet, dass** die Breite des spektralen Bandes, welchem der größte Teil der Fluoreszenz-Anregungsstrahlung für die DAFE II entstammt, nicht größer als 30 nm ist, dieses Band mindestens eine der Wellenlängen aus dem Bereich von 395 nm ± 5 nm einschließt und der größte Anteil derjenigen Strahlung, welche diesem Transmissionsband entstammt, durch eine Wellenlänge kleiner 400 nm charakterisiert ist.

4. Vorrichtung nach Anspruch 1 oder 2, **dadurch gekennzeichnet, dass** sich das spektrale Band, welchem der größte Teil der Fluoreszenz-Anregungsstrahlung für die DAFE II entstammt, im Langwelligen an das spektrale Band für die DAFE bzw. DAFE I anschließt, die Breite dieses spektralen Bandes weniger als 35 nm beträgt und die langwellige Kante dieses Bandes bei einer Wellenlänge von höchstens 445 nm liegt.

5. Vorrichtung nach einem der Ansprüche 1 bis 4, **dadurch gekennzeichnet, dass** die Strahlung aus dem spektralen Band, welches im Bereich der Wellenlänge von 405 nm liegt, durch Separierung der 405 nm- und 408 nm-Linie einer Gasentladungslampe mit Quecksilberanteilen mittels optischer Filtertechnik bereitgestellt wird.

6. Vorrichtung nach einem der Ansprüche 1 bis 4, **dadurch gekennzeichnet, dass** die Strahlung aus dem spektralen Band, welches im Bereich der Wellenlänge von 405 nm liegt, durch einen Laser oder eine Laserdiode oder einen Array von Laserdioden, welche im Bereich der Wellenlänge von 405 nm emittieren, bereitgestellt wird.

7. Vorrichtung nach einem der Ansprüche 1 bis 4, **dadurch gekennzeichnet, dass** die Strahlung aus dem spektralen Band, welches im Bereich der Wellenlänge von 405 nm liegt, durch eine Leuchtdiode oder einen Array von Leuchtdioden, welche im Bereich der Wellenlänge von 405 nm emittieren, bereitgestellt wird.

8. Vorrichtung nach einem der Ansprüche 1 bis 7, **dadurch gekennzeichnet, dass** es sich bei der Bildübertragungseinheit (4) um das Objektiv eines Videoendoskops, bei der Bildaufnahmeeinheit (5) um das Sensorsystem eines Videoendoskops, und bei der Bildverarbeitungseinheit (6) um den zugehörigen Controller handelt.

9. Vorrichtung nach einem der Ansprüche 1 bis 7, **dadurch gekennzeichnet, dass** es sich bei der Bildübertragungseinheit (4) um das Objektiv, das Linsensystem / Bildfaserbündel und das Okular eines Endoskops sowie um das Kameraobjektiv handelt und dass es sich bei der Bildaufnahmeeinheit (5) um das Sensorsystem einer Kamera und bei der Bildverarbeitungseinheit (6) um den zugehörigen Treiber handelt.
